**Europäisches Patentamt**

(19) **European Patent Office**

**Office européen des brevets**

(11) Publication number : **0 366 990 B1**

(12) # EUROPEAN PATENT SPECIFICATION

(45) Date of publication of patent specification :
**02.12.92 Bulletin 92/49**

(51) Int. Cl.⁵ : **A61K 31/41,** A61K 47/24, A61K 9/08

(21) Application number : **89118956.5**

(22) Date of filing : **12.10.89**

(54) **Stable parenteral solution of 2-phenyl-1.2-benzisoselenazol-3(2H)-one and process for producing the same.**

(30) Priority : **29.10.88 DE 3836892**

(43) Date of publication of application :
**09.05.90 Bulletin 90/19**

(45) Publication of the grant of the patent :
**02.12.92 Bulletin 92/49**

(84) Designated Contracting States :
**AT BE CH DE ES FR GB GR IT LI LU NL SE**

(56) References cited :
**EP-A- 0 044 971**
**EP-A- 0 165 534**

(73) Proprietor : **A. Nattermann & Cie. GmbH**
**Nattermannallee 1**
**W-5000 Köln 30 (DE)**

(72) Inventor : **Hager, Jörg**
**Hermann-Josef-Schmitt-Str. 48**
**W-5000 Köln 30 (DE)**
Inventor : **Hüther, Andrea Michaela**
**Brauweiler Weg 133**
**W-5000 Köln 41 (DE)**
Inventor : **Röding, Joachim**
**Weissenburger Str. 13**
**W-5000 Köln 1 (DE)**

(74) Representative : **Patentanwaltsbüro Cohausz**
**& Florack**
**Schumannstrasse 97**
**W-4000 Düsseldorf 1 (DE)**

EP 0 366 990 B1

## Description

The present invention is related to new solutions which may be parenterally administered and which comprise 2-phenyl 1.2-benzisoselenazol-3(2H)-one (Ebselen) in combination with one or several phospholipids preferably in a weight proportion of from 1:2500 to 1:15 and, possibly, one or several auxiliary agents. The invention is further related to the production of such solutions as well as their use in the production of drug preparations comprising 2-phenyl-1.2-benzisoselenazol-3(2H)-one (Ebselen) and one or several phospholipids.

Ebselen is a known product (DE-PS 3027073). It may be produced by the process of R. Weber and M. Renson, Bulletin de la Soc. Chim. de France 1976 (7/8), pgs. 1124-1126, by subjecting 2-methylseleno-N-phenyl-benzamide to reaction with phosphorous pentachloride and subsequently hydrolysing the obtained product. Preparations comprising Ebselen may be used in the treatment of numerous diseases such as the prophylaxis and therapy of infectious diseases, the therapy of malignant tumors (DE-OS 3638124), for stimulating the immune system or for the treatment of selenium deficiency diseases. Further attention is drawn to the application of the anti-arteriosclerotic and anti-inflammatory properties of Ebselen and their application in the therapy of rheumatic diseases (DE-OS 3027073). Ebselen is furthermore an important agent useful in the therapy of deficiencies caused by oxidative stress (DE-OS 3616920) such as liver deficiencies, cardiac infarction, psoriasis and radiation sicknen. There is known also a drug preparation for the topical use of Ebselen (DE-OS 3620674), which may be used in the external treatment of inflammatory and allergic skin diseases such as psoriasis.

The broad spectrum of properties is in contrast to a very low solubility of Ebselen in water. Due thereto the use of Ebselen in the form of parenteral solutions is prevented. Preparations comprising organic solvents containing Ebselen dissolved therein do not provide satisfactory results because diluting such solutions with water for injections or with physiological saline solutions cause precipitation of crystals of Ebselen.

It has now been found that surprisingly stable aqueous solutions of 2-phenyl-1.2-benzisoselenazol-3(2H)-one (Ebselen) having a physiological pH may be produced by combining Ebselen with preferably one or several natural or synthetic phospholipids and this in a weight proportion of Ebselen to phospholipid amounting to a ratio ranging from 1:2500 to 1:15. It required, further auxiliary agents may be added.

In this way, new aqueous solutions of Ebselen in combination with one or several phospholipids are formed. Such solutions are very suitable for parenteral administration (for instance for intramuscular or intravenous administration) and such solutions show a long duration of action.

For producing such solutions, the components thereof are added to each other and stirred to produce homogenous solutions in usual manners, for instance by aid of high pressure homogenators. In some instances it is possible to obtain the solutions by simple stirring. Another possibility to produce the solutions is treatment with ultrasonic methods or by using the so called "French Press".

One or more auxiliary agents, for example products to produce isotonic solutions, may be added before or after the preparation of the homogeneous solutions. Such products are for example sodium chloride or glucose. It may be advantageous to add a base, for instance soda lye or a buffer agent in order to produce a pH close to the physiological pH. The solutions thus prepared may be sterilized in usual manners and filled into ampoules as usual.

In view of the sensitivity of the phospholipids to light and oxygen, it may be preferable to work with the exclusion of oxygen and in an inert atmosphere, and with the exclusion of light.

Both natural and synthetic phospholipids may be used. Natural phospholipids (of plant or animal origin) are in particular phosphatidyl choline, phosphatidyl ethanolamine, phosphatidyl inositol, phosphatidyl glycol, cardiolipine or plasmalogens, which products may be recovered from soybeans or from eggs. Examples of natural phosphatides are soybean lecithin and egg lecithin and highly purified mixtures thereof. Further useful are mixtures of several such phospholipids such as the trade products

Phospholipon (R) 100        (95% natural phosphatidyl choline from soybeans)
Phospholipon (R) 100 H      (98% fully hydrogenated phosphatidyl choline from soybeans)
Phospholipon (R) 80         (phospholipids from soybeans comprising 76% of phosphatidyl choline and 12% of phosphatidyl ethanolamine).

Synthetic phosphatides are for instance:
dihexadecanoylphosphatidylcholine,
ditetradecanoylphosphatidylcholine,
dioleylphosphatidylcholine,
dilinolylphosphatidylcholine,
in particular
dipalmitoylphosphatidylcholine and
dipalmitoylphosphatidylglycerol.

Auxiliary agents are for instance cholesterol, derivatives of bile acids and salts thereof, benzyl alcohol, neutral oils (Miglyol 812) and glycerol. (Miglyol [R] is a trademark of Messrs. Dynamit Nobel AG of Troisdorf/Cologne, Germany.)

The production of the preparations according to the present invention is further illustrated in the following examples.

Example 1

| Ebselen | 0.110 g |
| DPPC (dipalmitoylphosphatidylcholine) | 13.330 g |
| DPPG (dipalmitoylphosphatidylglycerol) | 1.330 g |
| cholesterol | 6.450 g |
| buffering agent to pH4 | up to 1000 ml |

Ebselen, DPPC, DPPG and cholesterol are dissolved in a mixture of 1 part of methanol and 1 part of chloroform. The solvent is removed and the resulting film is hydrated with buffer under an inert gas. Glass beads are added and liposomes are formed with stirring. They are filtered in usual manner under sterile conditions and filled into ampoules.

Example 2

| Ebselen | 0.150 g |
| DPPC | 18.180 g |
| DPPG | 1.818 g |
| cholesterol | 8.790 g |
| water for injections | up to 1000 ml |

The products are mixed and further processed as described in Example 1.

Example 3

| Ebselen | 0.250 g |
| DPPC | 30.300 g |
| DPPG | 3.030 g |
| cholesterol | 14.650 g |
| buffering agent | up to 1000 ml |

The products are mixed and further processed as described in Example 1.

Example 4

| Ebselen | 0.330 g |
| DPPC | 39.970 g |
| DPPG | 3.997 g |
| cholesterol | 19.338 g |
| buffering agent | up to 1000 ml |

The products are mixed and further processed as described in Example 1.

Example 5

| Ebselen | 0.400 g |
| DPPC | 48.480 g |
| DPPG | 4.848 g |
| cholesterol | 23.440 g |
| buffering agent | up to 1000 ml |

The products are mixed and further processed as described in in Example 1.

Example 6

| | |
|---|---|
| Ebselen | 0.430 g |
| DPPC | 50.170 g |
| DPPG | 5.017 g |
| cholesterol | 24.112 g |
| buffering agent | up to 1000 ml |

The products are mixed and further processed as described in in Example 1.

Example 7

| | |
|---|---|
| Ebselen | 0.100 g |
| Phospholipon (R) 100 | 45.215 g |
| sodiumdesoxycholate | 17.621 g |
| benzyl alcohol | 15.700 g |
| water for injections | up to 1000 ml |

Ebselen and Phospholipon 100 are dissolved in ethanol. After removal of the solvent under vacuum, the resulting mixture is stirred into a solution of sodium desoxycholate. After the addition of benzyl alcohol and water as described in Example 1, the solution is filtered under sterile conditions and filled into ampoules.

Example 8

| | |
|---|---|
| Ebselen | 0.300 g |
| Phospholipon (R) 100 | 116.900 g |
| sodium desoxycholate | 45.900 g |
| benzyl alcohol | 15.700 g |
| water for injections | up to 1000 ml |

The products are mixed and further processed as described in in Example 7.

Example 9

| | |
|---|---|
| Ebselen | 0.300 g |
| Phospholipon (R)100 | 116.900 g |
| glycocholic acid | 58.200 g |
| NaOH | 5.000 g |
| benzyl alcohol | 15.700 g |
| water for injections | up to 1000 ml |

Example 10

| | |
|---|---|
| Ebselen | 0.300 g |
| Phospholipon (R) 80 | 116.900 g |
| imrocholic acid | 64.370 g |
| NaOH | 5.000 g |
| benzyl alcohol | 15.700 g |
| water for injections | up to 1000 ml |

The products are mixed and further processed as described in in Example 7.

Example 11

| | |
|---|---|
| Ebselen | 0.450 g |
| Phospholipon (R) 100 | 110.440 g |
| sodium cholate | 40.125 g |
| benzyl alcohol | 15.700 g |
| water for injections | up to 1000 ml |

The products are mixed and further processed as described in in Example 7.

Example 12

| | |
|---|---|
| Ebselen | 0.500 g |
| Phospholipon (R) 80 | 108.700 g |
| water for injections | up to 1000 ml |

Ebselen and Phospholipon (R) 100 are dispersed with stirring in water for injection purposes. The resulting mixture is treated in the high pressure homogeniser. The further subsequent filtration under sterile conditions and filling into ampoules is executed as descried in Example 1.

Example 13

| | |
|---|---|
| Ebselen | 0.420 g |
| Phospholipon (R) 80 | 111.250 g |
| water for injections | up to 1000 ml |

The products are mixed and further processed as described in in Example 12.

Example 14

| | |
|---|---|
| Ebselen | 0.200 g |
| lecithin | 20.000 g |
| Miglyol® 812 | 170.000 g |
| glycerol | 16.000 g |
| water for injections | up to 1000 ml |

Ebselen is dissolved in Miglyol 812 and lecithine (solution I). Glycerol is added to the water for injection purposes (solution II). Both solutions are mixed and treated in the high pressure homogeniser. The resulting emulsion is sterilised in an autoclave and filled into ampoules as usual.

Example 15

| | |
|---|---|
| Ebselen | 0.500 g |
| lecithin | 24.000 g |
| Miglyol® 812 | 200.000 g |
| glycerol | 32.000 g |
| water for injections | up to 1000 ml |

The products are mixed and further processed as described in in Example 14.

Example 16

| | |
|---|---|
| Ebselen | 1.000 g |
| lecithine | 24.000 g |
| Miglyol® 812 | 200.000 g |
| glycerol | 32.000 g |
| water for injections | up to 1000 ml |

The products are mixed and further processed as described in in Example 14.

Example 17

| | |
|---|---|
| Ebselen | 2.000 g |
| lecithine | 32.000 g |
| Miglyol® 812 | 220.000 g |
| glycerol | 37.000 g |
| water for injections | up to 1000 ml |

The products are mixed and further processed as described in in Example 14.

## Claims

### Claims for the following Contracting States : AT, BE, CH, DE, FR, GB, IT, LI, LU, NL, SE

1. Stable parenteral solutions of 2-phenyl-1.2-benzisoselenazol-3(2H)-one, characterized in that they contain a water-soluble combination of 2-phenyl-1.2-benzisoselenazol-3(2H)-one with one or several phospholipids.

2. Solution according to claim 1, characterized in that the weight proportion of 2-phenyl-1.2-benzisoselenazol-3(2H)-one and the phospholipid or phospholipids in the solution is between 1:2500 to 1:15.

3. Solution according to claim 1 or 2, characterized in that the phospholipid or the phospholipids are natural or synthetic phospholipids.

4. Solution according to claims 1 to 3, characterized in that it comprises as natural phospholipid the compound soybean lecithin or egg lecithine or a highly purified fraction thereof.

5. Solution according to claims 1 to 3, characterized in that the natural phospholipid is phosphatidyl choline, phosphatidyl glycerol or a mixture thereof.

6. Process for producing drug preparations as claimed in claims 1 to 5, characterized in that 2-phenyl-1.2-benzisoselenazol-3(2H)-one (Ebselen) is mixed with the phospholipid or a mixture of several phospholipids and, possibly, one or several additional auxiliary agents in known manner, the mixture is added to a physiological saline solution, the resulting physiological solution is rendered neutral, possibly by means of additional auxiliary agents, and the resulting solution is rendered sterile.

7. The use of solutions according to any of claims 1 to 5 comprising a combination of 2-phenyl-1.2-benzisoselenazol-3(2H)-one (Ebselen) and one or several phospholipids and, possibly, one or several auxiliary agents, for the preparation of drugs.

### Claims for the following Contracting States : ES, GR

1. Process for producing stable parenteral solutions of 2-phenyl-1.2-benzisoselenazol-3(2H)-one, characterized in that 2-phenyl-1.2-benzisoselenazol-3(2H)-one (Ebselen) is mixed with a phospholipid or a mixture of several phospholipids and, possibly, one or several additional auxiliary agents in known manner, the mixture is added to a physiological saline solution, the resulting physiological solution is rendered neutral, possibly my means of additional auxiliary agents, and the resulting solution is rendered sterile.

2. Process according to claim 1, characterized in that the weight proportion of 2-phenyl-1.2-benzisoselenazol-3(2H)-one and the phospholipid or phospholipids in the solution is between 1:2500 to 1:15.

3. Process according to one or both of claims 1 and 2, characterized in that the phospholipid or the phospholipids are natural or synthetic phospholipids.

4. Process according to one or several of claims 1 to 3, characterized in that it comprises as natural phospholipid the compound soybean lecithin or egg lecithin or a highly purified fraction thereof.

5. Process according to one or several of claims 1 to 4, characterized in that the natural phospholipid is phosphatidyl choline, phosphatidyl glycerol or a mixture thereof.

## Patentansprüche

### Patentansprüche für folgende Vertragsstaaten : AT, BE, CH, DE, FR, GB, IT, LI, LU, NL, SE

1. Stabile, parenteral applizierbare Lösungen von 2-Phenyl-1,2-benzisoselenazol-3(2H)-on, dadurch gekennzeichnet, daß sie eine wasserlösliche Kombination aus 2-Phenyl-1,2-benzisoselenazol-3(2H)-on mit einem oder mehreren Phospholipiden enthalten.

**2.** Lösungen nach Anspruch 1, dadurch gekennzeichnet, daß das Gewichtsverhältnis von 2-Phenyl-1,2-benzisoselenazol-3(2H)-on und dem oder den Phospholipiden in der Lösung 1:2500 bis 1:15 beträgt.

**3.** Lösungen nach Anspruch 1 oder 2, dadurch gekennzeichnet, daß sie als das oder die Phospholipide natürliche oder synthetische Phospholipide enthalten.

**4.** Lösungen nach den Ansprüchen 1 bis 3, dadurch gekennzeichnet, daß sie als natürliches Phospholipid Sojalecithin oder Ei-Lecithin oder eine hoch gereinigte Fraktion derselben enthalten.

**5.** Lösungen nach den Ansprüchen 1 bis 3, dadurch gekennzeichnet, daß sie als natürliches Phospholipid Phosphatidylcholin, Phosphatidylglycerol oder Mischungen hiervon enthalten.

**6.** Verfahren zur Herstellung von Arzneimitteln nach den Ansprüchen 1 bis 5, dadurch gekennzeichnet, daß man 2-Phenyl-1,2-benzisoselenazol-3(2H)-on (Ebselen) und das Phospholipid oder ein Gemisch mehrerer Phospholipide und ggfs. ein oder mehrere Hilfsmittel nach üblichen Methoden miteinander vermischt, die Mischung zu einer physiologischen Lösung gibt, die physiologische Lösung auf einen neutralen pH-Wert - ggfs. mit weiteren Hilfsstoffen - einstellt und in eine sterile, stabile Lösung überführt.

**7.** Verwendung von Lösungen nach den Ansprüchen 1 bis 5 mit 2-Phenyl-1,2-benzisoselenazol-3(2H)-on (Ebselen) und ein oder mehrere Phsopholipiden sowie ggfs. einem oder mehreren Hilfsstoffen zur Herstellung von Arzneizubereitungen.

**Patentansprüche für folgende Vertragsstaaten : ES, GR**

**1.** Verfahren zur Herstellung stabiler, parenteraler Lösungen von 2-Phenyl-1,2-benzisoselenazol- 3(2H)-on, dadurch gekennzeichnet, daß
2-Phenyl-1,2-benzisoselenazol-3(2H)-on (Ebselen) mit einem Phospholipid oder einer Mischung aus mehreren Phospholipiden und gegebenenfalls einem oder mehreren Hilfsmitteln nach üblichen Methoden miteinander vermischt wird, die Mischung zu einer physiologischen Kochsalzlösung zugegeben wird, die resultierende physiologische Lösung auf einen neutralen pH-Wert - ggfs. mit weiteren Hilfsstoffen - einstellgestellt wird und die resultierende Lösung sterilisiert wird.

**2.** Verfahren gemäß Anspruch 1, dadurch gekennzeichnet, daß das Gewichtsverhältnis von 2-Phenyl-1,2-benzisoselenazol-3(2H)-on zu dem oder den Phospholipiden in der Lösung 1:2500 bis 1:15 ist.

**3.** Verfahren nach einem oder beiden der Ansprüche 1 und 2, dadurch gekennzeichnet, daß das oder die Phospholipide ein natürliches oder synthetisches Phsopholipid sind.

**4.** Verfahren nach einem oder mehreren der Ansprüche 1 bis 3, dadurch gekennzeichnet, daß als natürliches Phospholipid Sojabohnenlecithin oder Ei-Lecithin oder eine hoch gereinigte Fraktion hiervon ist.

**5.** Verfahren gemäß einem oder mehreren der Ansprüche 1 bis 4, dadurch gekennzeichnet, daß das natürliche Phospholipid Phosphatidylcholin, Phsophatidylglycerol oder ein Gemisch von beiden ist.

**Revendications**

**Revendications pour les Etats contractants suivants : AT, BE, CH, DE, FR, GB, IT, LI, LU, NL, SE**

**1.** Solutions stables parentérales de 2-phényl-1,2-benzisosélénazole-3(2H)-one, caractérisées en ce qu'elles contiennent une combinaison soluble dans l'eau de 2-phényl-1,2-benzisosélénazole-3(2H)-one avec un ou plusieurs phospholipides.

**2.** Solution selon la revendication 1, caractérisée en ce que la proportion en poids de la 2-phényl-1,2-benzisosélénazole-3(2H)-one et du phospholipide ou des phospholipides dans la solution est située entre 1:2500 et 1:15.

**3.** Solution selon la revendication 1 ou 2, caractérisée en ce que le phospholipide ou les phospholipides sont des phospholipides naturels ou synthétiques.

4. Solution selon les revendications 1 à 3, caractérisée en ce qu'elle comporte comme phospholipide naturel le composé formé par la lécithine du soja ou la lécithine de l'oeuf ou une fraction de celles-ci très purifiée.

5. Solution selon les revendications 1 à 3, caractérisée en ce que le phospholipide naturel est la phosphatidyl choline, le phosphatidyl glycérol, ou un mélange de ceux-ci.

6. Procédé pour préparer des préparations de médicaments telles que revendiquées dans les revendications 1 à 5, caractérisé en ce que !a 2-phényl-1,2-benzisosélénazole-3(2H)-one (Ebselen) est mélangée avec le phospholipide, ou un mélange de plusieurs phospholipides, et, éventuellement, un ou plusieurs agents auxiliaires additionnels de façon connue, le mélange est ajouté à une solution physiologique saline, la solution physiologique résultante est rendue neutre, éventuellement au moyen d'agents auxiliaires additionnels, et la solution résultante est rendue stérile.

7. Emploi de solutions conformes à l'une quelconque des revendications 1 à 5, comportant une combinaison de la 2-phényl-1,2-benzisosélénazole-3(2H)-one (Ebselen) et d'un ou plusieurs phospholipides, et, éventuellement, d'un ou plusieurs agents auxiliaires, pour la préparation de médicaments.

**Revendications pour les Etats contractants suivants : ES, GR**

1. Procédé pour produire des solutions parentérales stables de 2-phényl-1,2-benzisosélénazole-3(2H)-one, caractérisé en ce que la 2-phényl-1,2-benzisosélénazole-3(2H)-one (Ebselen) est mélangée avec un phospholipide ou un mélange de plusieurs phospholipides, et, éventuellement un ou plusieurs agents auxiliaires additionnels de façon connue, le mélange est ajouté à une solution physiologique saline , la solution physiologique résultante est rendue neutre, éventuellement au moyen d'agents auxiliaires additionnels, et la solution résultante est rendue stérile.

2. Procédé selon la revendication 1, caractérisé en ce que la proportion en poids de la 2-phényl-1,2-benzisosélénazole-3(2H)-one et du phospholipide ou des phospholipides dans la solution est compris entre 1:2500 et 1:15.

3. Procédé selon l'une ou les deux des revendications 1 et 2, caractérisé en ce que le phospholipide ou les phospholipidses sont des phospholipides naturels ou synthétiques.

4. Procédé selon l'une ou plusieurs des revendications 1 à 3, caractérisé en ce qu'il comporte comme phospholipide naturel le composé formé par la lécithine du soja ou la lécithine de l'oeuf ou une fraction très purifiée de celles-ci.

5. Procédé selon l'une ou plusieurs des revendications 1 à 4, caractérisé en ce que le phospholipide naturel est la phosphatidyl choline, le phosphatidyl glycérol ou un mélange de ceux-ci.